# EUROPEAN PATENT APPLICATION

(11) **EP 2 517 625 A1**
(43) Date of publication of application: **31.10.2012**
(21) Application number: 10839128.5
(22) Date of filing: 25.11.2010
(51) Int. Cl.: A61B 5/1486

(54) **MEASUREMENT DEVICE AND SENSOR PLACEMENT METHOD**

(30) Priority: 24.12.2009 JP 2009291706; 29.01.2010 JP 2010017723
(71) Applicant: Arkray, Inc., Kyoto-shi, Kyoto 601-8045 (JP)
(72) Inventor: TSUKADA, Masashi, Kyoto-shi Kyoto 6020008 (JP); KUSAKA, Yasuhide, Kyoto-shi Kyoto 6020008 (JP); YAMAMOTO, Akihiro, Kyoto-shi Kyoto 6020008 (JP)
(74) Representative: MacDougall, Alan John Shaw
(86) International application number: PCT/JP2010/071000
(87) International publication number: WO 2011/077893

(57) **Abstract**

Provided are a measurement device, a sensor unit, and a sensor placement method using those, which enable the suppression of the occurrence of a situation in which when a measurement is carried out with a sensor implanted under the skin, the implanted sensor becomes faulty. A sensor unit (2) is used for measuring numerical information relating to a substance contained in a body fluid in a body The sensor unit (2) is provided with a sensor (15) a part of which is placed under the skin and generates a signal according to the state of the substance, a base (10) which is disposed on the skin and holds the sensor (15), a variable mechanism (11) which is attached to the base (10) and enables the position and/or the direction of the sensor (15) with reference to the base (10) to be varied, and an external terminal (12) which is provided in the base (10) and leads the signal generated by the sensor (15) to the outside.

## Description

### Technical Field

The present invention relates to a measuring apparatus and a sensor placement method that are for measuring numerical information relating to a substance contained in interstitial fluid or blood, and particularly for measuring glucose concentration.

### Background Art

With conventional blood sugar level measurement, it is necessary to puncture the patient's body with an instrument called a lancet and take a blood sample whenever measurement is carried out, and thus there is a problem in that a large burden is placed on the patient, and, furthermore, continuous measurement cannot be carried out. In order to solve such problems, a method of continuously measuring glucose concentration in subcutaneous tissue called CGM (Continuous Glucose Monitoring) has been proposed in recent years.

With CGM, a sensor is disposed so as to be partially embedded under the patient's skin, and the signal of a current value or the like that depends on the concentration of glucose in subcutaneous interstitial fluid is continuously output by this sensor. The signal is then converted to a blood sugar level by a measurement apparatus or the like. CGM enables blood sugar levels to be measured continuously (e.g., see Patent Document 1). Although interstitial fluid differs from blood, it is thought that the concentration of glucose in interstitial fluid reflects the concentration of glucose (blood sugar level) in blood. Therefore, blood sugar levels can be known by measuring the concentration of glucose in subcutaneous interstitial fluid.

Also, generally, the sensor, in order to be able to flexibly deal with the body movement of muscles and the like under the skin, is constituted by a flexible strip-like substrate or linear wire. In the case of the former, a sensor electrode that outputs a signal, a terminal for external connection, and wiring that connects the sensor electrode and the external connection terminal are formed on the substrate (e.g., see Patent Document 1).

Furthermore, since CGM requires that the sensor be partially implanted under the patient's skin, Patent Document 1 discloses a device (implanting device) that is able to drive out the sensor toward the skin together with a puncture needle, and implant the sensor under the skin. The implanting device is provided with a mechanism that drives out the sensor together with the puncture needle using a spring or the like, and thereafter pulls back only the puncture needle. Here, the procedure for inserting the sensor disclosed in Patent Document 1 is described.

First, a mount unit for mounting the sensor is disposed on the patient's skin. The implanting device in which the sensor and the puncture needle are set is then disposed on a prescribed position of the mount unit, and the sensor and the puncture needle are both driven under the skin by the implanting device. Thereafter, the puncture needle returns to its original position, and the sensor is disposed with the portion on which a terminal for connection is provided projecting above the skin and the remaining portion placed under the skin.

When the implanting device has been removed from the mount unit, a control unit for controlling the sensor is disposed on the mount unit. At this time, the portion of the sensor on which in the terminal is provided (terminal portion) is sandwiched between the mount unit and the control unit, and, at the same time, the terminal of the control unit and the terminal of the sensor that projects above the skin are connected.

If sensing by the sensor is performed in this state, the signal obtained by the sensor is converted to a digital signal by the control unit, and is furthermore sent to an external measurement apparatus by wireless or cable. The measurement apparatus calculates the specific concentration of glucose from the received signal, and displays the calculated value on a display screen.

### Citation List

### Patent Documents

Patent Document 1: JP 2008-62072A (FIG. 11, FIG. 14, FIGS. 26-28D, FIG. 33)

### Disclosure of the Invention

### Problem to be Solved by the Invention

Incidentally, while the terminal portion of the sensor is, as mentioned above, sandwiched between the mount unit and the control unit in order to connect the terminal of the sensor and the terminal of the control unit, the sensor needs to be elastically deformed at this time. If the portion of the sensor embedded under the skin moves when the sensor is elastically deformed, the wound formed in the skin by the implanting device becomes bigger.

In such a case, since the body covers the sensor in protein in order to heal the wound, the sensor may not be able to output a signal, or a signal may be output but include noise, thus preventing accurate measurement. Furthermore, since the sensor thus covered in protein cannot be used, it needs to be removed and a new sensor reinserted, placing a not insignificant physically and financial burden on the patient. Since it is only when the control unit and the measurement apparatus are operated that it first becomes evident whether or not the sensor is outputting a signal, the case may also arise where the patient has to visit a medical facility again.

Also, while the sensor disclosed in Patent Document 1 is, as mentioned above, connected to the control unit by the terminal portion exposed outside the body after being placed under the skin, this connection process is performed by the user himself or herself (see FIG. 14 of Patent Document 1). For this reason, situations may occur where human operational error at the time of connection results in a load being placed on the portion of the sensor inserted under the skin or the wound formed in the insertion site being made bigger.

Since the body also covers the sensor in protein in these cases in order to heal the wound, the sensor may not be able to output a signal or a signal may be output but include noise, thus preventing accurate measurement. Furthermore, since a new sensor needs to be reinserted, the physically and financial burden placed on the patient is not insignificant. The case may also arise where the patient has to visit a medical facility again.

Also, given that the sensor has, for example, a full length of several centimeters and a width of several millimeters, or is smaller in size than this, the external connection terminal of the sensor and the terminal of the control unit are minute. For this reason, a poor connection may occur between the sensor and the control unit during the above-mentioned connection process by the user. Furthermore, the substrate on which the external connection terminal of the sensor is formed may also move due to movement of the body such as intense physical activity, also resulting in a poor connection between the sensor and the control unit. In the case where a poor connection such as this arises, the signal from the sensor is not transmitted to the control unit or, moreover, to the measurement apparatus, rendering measurement impossible.

An exemplary object of the present invention is to solve the above-mentioned problems, and to provide a measuring apparatus and a sensor placement method that enable situations where the function of an embedded sensor is impaired when embedding the sensor under the skin and performing measurement to be suppressed.

### Means for Solving the Problem

In order to attain the above-mentioned object, the first measuring apparatus of the present invention is a measuring apparatus for measuring numerical information relating to a substance contained in a body fluid within a body that includes a sensor unit and a control unit, the sensor unit including a sensor that generates a signal according to a state of the substance, a base that holds the sensor, and a variable mechanism that is attached to the base and enables at least one of a position and an orientation of the sensor to be changed, and the control unit being formed so as to be attachable to the base and executing processing after receiving the signal generated by the sensor.

With the first measuring apparatus in the present invention, the base and the sensor are thus attached via the variable mechanism. Thus, even if the base moves when attaching the control unit, external force generated thereby is absorbed by the variable mechanism and the occurrence of a situation where the sensor itself moves is suppressed. Furthermore, even if stress such as jarring or twisting occurs due to physical activity when the patient is wearing the sensor, the influence exerted on the embedded sensor is reduced. Thus, according to the first measuring apparatus of the present invention, the occurrence of a situation where the function of an embedded sensor is impaired when embedding the sensor under the skin and performing measurement is suppressed.

Also, the first measuring apparatus of the present invention may adopt a mode in which the variable mechanism includes a ball joint, and a shaft at one end of the ball joint is attached to the sensor and a shaft at the other end of the ball joint is attached to the base. Furthermore, the first measuring apparatus of the present invention may adopt a mode in which the variable mechanism includes a rotating member that is held in a rotatable state, and the rotating member is attached to the sensor. These modes enable external force to be efficiently absorbed with a simple configuration.

Also, in order to attain the above-mentioned object, a second measuring apparatus of the present invention is a measuring apparatus for measuring numerical information relating to a substance contained in a body fluid within a body that includes a sensor unit and a control unit, the sensor unit including a sensor that generates a signal according to a state of the substance, a base that holds the sensor, and an external terminal that is provided in the base and directs the signal generated by the sensor to the outside, and the control unit being formed so as to be attachable to the base and executing processing after receiving the signal generated by the sensor via the external terminal.

According to the second measuring apparatus in the present invention, the sensor is thus connected to the control unit via the external terminal provided in the base. For this reason, the load placed on the portion of the sensor inserted within the body (e.g., under the skin) when connecting the sensor and the control unit is reduced. Also, a poor connection between the sensor and the control unit is less likely to occur. As a result, using the measuring apparatus, the sensor unit, and sensor placement apparatus of the present invention enables the occurrence of a loss of sensor function or a situation where measurement cannot be performed when embedding a sensor within the body and performing measurement to be suppressed.

Also, the second measuring apparatus of the present invention preferably adopts a mode in which the sensor unit further includes a variable mechanism that is attached to the base and enables at least one of a position and an orientation of the sensor to be changed. With this mode, because external force exerted on the sensor and the control unit when they are being connected is absorbed by the variable mechanism, the occurrence of a loss of sensor function is further suppressed.

Also, in order to attain the above-mentioned object, a first sensor placement method of the present invention is a method for placing a sensor within a body, the sensor generating a signal according to a state of a substance contained in a body fluid within the body, the method including the steps of (a) disposing a base on skin, the base being provided with an external terminal that directs the signal generated by the sensor to the outside, (b) partially implanting the sensor within the body, and causing the sensor to be held by the base, and (c) attaching a control unit to the base, the control unit executing processing after receiving the signal generated by the sensor via the external terminal.

With the above first sensor placement method, a variable mechanism that enables at least one of a position and an orientation of the sensor to be changed is attached to the base.

The first sensor placement method may adopt a mode in which the variable mechanism includes a ball joint, and a shaft at one end of the ball joint is attached to the sensor and a shaft at the other end of the ball joint is attached to the base.

Also, the first sensor placement method of the above may adopt a mode in which the variable mechanism includes a rotating member that is held in a rotatable state, and the rotating member is attached to the sensor.

Furthermore, the first sensor placement method of the above may adopt a mode in which the step (b) comprises partially implanting the sensor within the body, at the same time as which the base and the sensor become electrically connected.

Also, in order to attain the above-mentioned object, a second sensor placement method of the present invention is a method for placing a sensor within a body, the sensor generating a signal according to a state of a substance contained in a body fluid within the body, the method including the steps of (a) disposing a base on skin in a state where the sensor is held by the base, and partially implanting the sensor within the body, and (b) attaching a control unit to the base, the control unit executing processing after receiving the signal generated by the sensor.

With the above second sensor placement method, a variable mechanism that enables at least one of a position and an orientation of the sensor to be changed may be attached to the base. In this case, a mode may be adopted in which the variable mechanism includes a ball joint, and a shaft at one end of the ball joint is attached to the sensor and a shaft at the other end of the ball joint is attached to the base. Also, a mode may be adopted in which the variable mechanism includes a rotating member that is held in a rotatable state, and the rotating member is attached to the sensor.

Furthermore, in the above second sensor placement method, an external terminal that directs the signal generated by the sensor to the outside may be provided in the base, and the control unit may include a terminal that contacts with the external terminal included in the base. In this case, the step (b) comprises connecting the external terminal provided in the base and the terminal included in the control unit.

### Effects of the Invention

As described above, a measuring apparatus and a sensor placement method of the present invention enable the occurrence of situations where the performance of an embedded sensor deteriorates when embedding the sensor under the skin and performing measurement to be suppressed.

### Brief Description of Drawings

FIG. 1 is a perspective view showing configurations of a measuring apparatus and a sensor unit in Embodiment 1 of the present invention.
FIG. 2 is a perspective view showing a tip portion of the sensor shown in FIG. 1.
FIG. 3A and FIG. 3B are diagrams showing a series of steps of a sensor placement method in Embodiment 1 of the present invention.
FIG. 4A and FIG. 4B are diagrams showing a series of steps of the sensor placement method in Embodiment 1 of the present invention, these steps being executed after execution of the step shown in FIG. 3B.
FIG. 5 is a cross-sectional view showing an exemplary schematic configuration of an implanting device used in implementation of the sensor placement method in Embodiment 1 of the present invention.
FIG. 6 is a perspective view showing a first exemplary configuration of a sensor unit in Embodiment 2 of the present invention.
FIG. 7 is a perspective view showing a second exemplary configuration of the sensor unit in Embodiment 2 of the present invention.
FIG. 8 includes perspective views showing a configuration of a sensor unit in Embodiment 3 of the present invention, FIG. 8A showing a state where the sensor is removed and FIG. 8B showing a state where the sensor is attached.
FIG. 9A and FIG. 9B are diagrams showing a series of steps of a sensor placement method in Embodiment 2 of the present invention.
FIG. 10A and FIG. 10B are diagrams showing a series of steps of the sensor placement method in Embodiment 2 of the present invention, these steps being executed after execution of the step shown in FIG. 9B.
FIG. 11 is a perspective view showing a configuration of a measuring apparatus in Embodiment 4 of the present invention.
FIG. 12A and FIG. 12B are diagrams showing a series of steps of a sensor placement method in Embodiment 4 of the present invention.
FIG. 13A and FIG. 13B are diagrams showing a series of steps of the sensor placement method in Embodiment 4 of the present invention, these steps being executed after execution of the step shown in FIG. 12B.
FIG. 14 includes perspective views showing a configuration of a sensor unit in Embodiment 5 of the present invention, FIG. 14A showing a state where the sensor is removed and FIG. 14B showing a state where the sensor is attached.
FIG. 15A and FIG. 15B are diagrams showing a series of steps of a sensor placement method in Embodiment 5 of the present invention.
FIG. 16A and FIG. 16B are diagrams showing a series of steps of the sensor placement method in Embodiment 5 of the present invention, these steps being executed after execution of the step shown in FIG. 15B.

### Best Mode for Carrying Out the Invention

### Embodiment 1

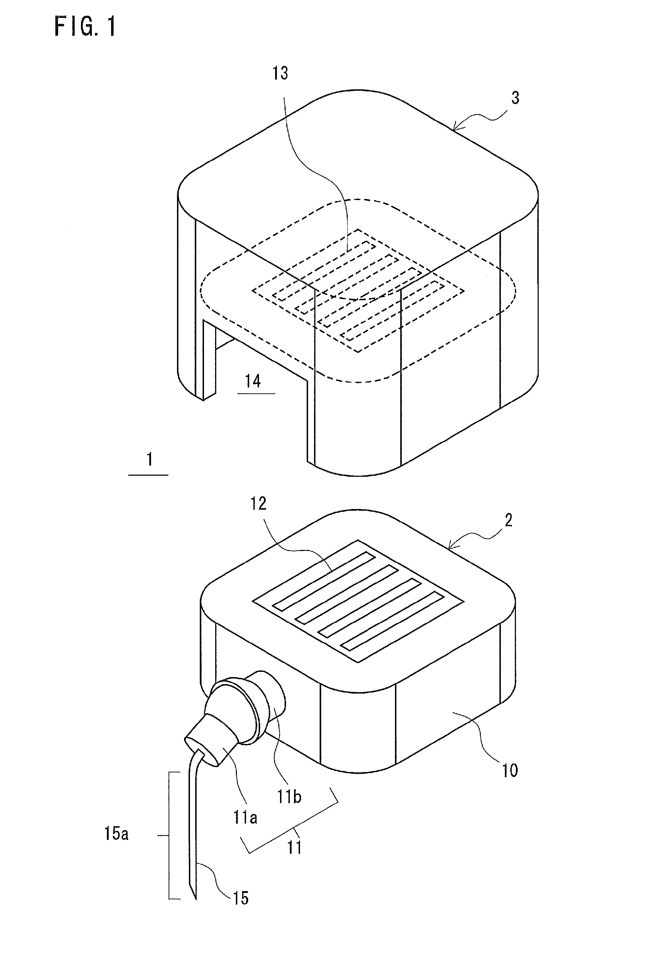

Hereinafter, a measuring apparatus and a sensor placement method in Embodiment 1 of the present invention are described, with reference to FIG. 1 to FIG. 3. Initially, configurations of a measuring apparatus 1 and a sensor unit 2 in the present Embodiment 1 are described using FIG. 1. FIG. 1 is a perspective view showing the configurations of the measuring apparatus and the sensor unit in Embodiment 1 of the present invention.

The measuring apparatus 1 shown in FIG. 1 is an apparatus that measures numerical information relating to a substance contained in a body fluid within the body. As shown in FIG. 1, the measuring apparatus 1 is provided with the sensor unit 2 and a control unit 3. Note that examples of body fluid within the body include interstitial fluid, blood and plasma. Furthermore, in the present specification, "within the body" includes "under the skin" indicating below the skin surface.

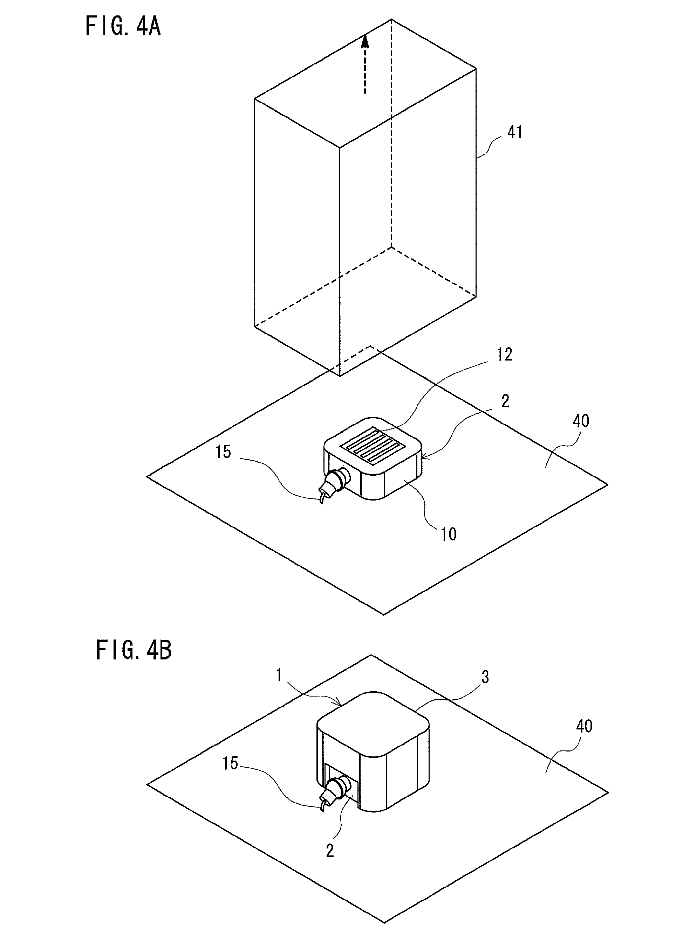

The sensor unit 2 is provided with a base 10, a variable mechanism 11, and a sensor 15. Of these, the sensor 15 is placed partially under the skin, in order to execute CGM (see FIG. 4A and FIG. 4B discussed below). The sensor unit 2 will also function as a sensor placement apparatus for placing the sensor 15. Also, the sensor 15 generates a signal according to the state of a substance in interstitial fluid or blood.

The base 10 is disposed on the skin of the patient who is being measured and holds the sensor 15. The variable mechanism 11 is attached to the base 10, and interposes between the base 10 and the sensor 15. Also, the variable mechanism 11 enables at least one of a position and an orientation of the sensor 15 to be changed based on the base 10. Note that the position and the orientation of the sensor 15 based on the base 10 denotes the relative position and relative orientation of the sensor to the base.

The control unit 3 receives the signal generated by the sensor 15 via an external terminal 12, and executes processing based on the received signal. Also, the control unit 3 is formed so as to be attachable to the base 10.

In the present Embodiment 1, the sensor 15 is thus held by the base 10 via the variable mechanism 11. Therefore, even if the control unit 3 is attached in a state where the sensor 15 is partially embedded and the base 10 moves at that time, the external force generated thereby is absorbed by the variable mechanism 11, preventing the sensor 15 itself from moving.

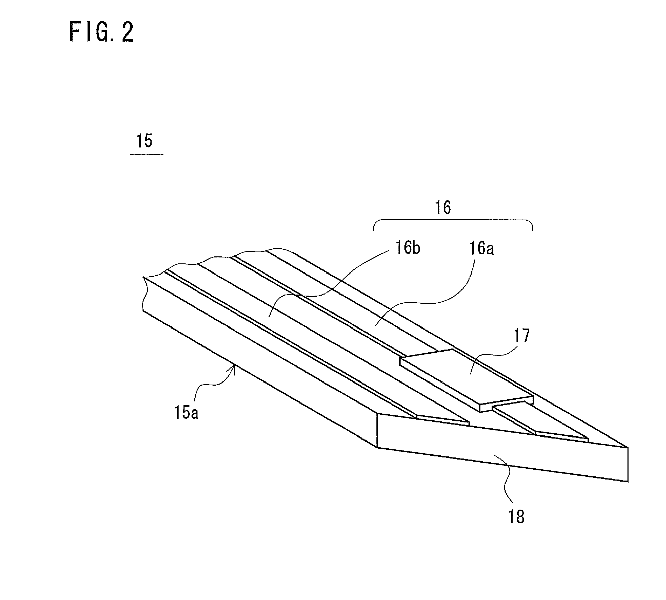

Here, configurations of the measuring apparatus 1 in the present Embodiment 1 and the sensor unit 2 and the control unit 3 constituting the measuring apparatus 1 are more specifically described using FIG. 2. FIG. 2 is a perspective view showing a tip portion of the sensor shown in FIG. 1.

In the present Embodiment 1, the sensor unit 2 includes the external terminal 12. The external terminal 12 is provided in the base 10. Also, the external terminal 12 is electrically connected to the sensor 15 as discussed later, enabling the signal generated by the sensor 15 to be directed to the outside. Furthermore, the control unit 3 receives the signal generated by the sensor 15 via the external terminal 12. In the present Embodiment 1, the signal generated by the sensor 15 is sent to the control unit 3 via the external terminal 12 provided in the base 10.

Also, in the present Embodiment 1, the substance that is measured is glucose in interstitial fluid, and the numerical information relating to the substance is the concentration of glucose. The sensor 15 generates a signal according to the state (concentration) of glucose in interstitial fluid. In the following, an example is described in which the numerical information relating to the substance is the concentration of glucose, and the sensor 15 is a glucose sensor. Note that in the present Embodiment 1, the substance that is measured may be a substance other than glucose, and may be a substance in blood. Also, the numerical information may be information other than concentration.

Also, in the present Embodiment 1, the sensor 15 is able to continuously output a signal that depends on the state of glucose in interstitial fluid, and allow the measuring apparatus 1 to function as a monitoring apparatus capable of continuously monitoring the concentration of glucose. In this case, the measuring apparatus 1 is able to perform the above-mentioned CGM.

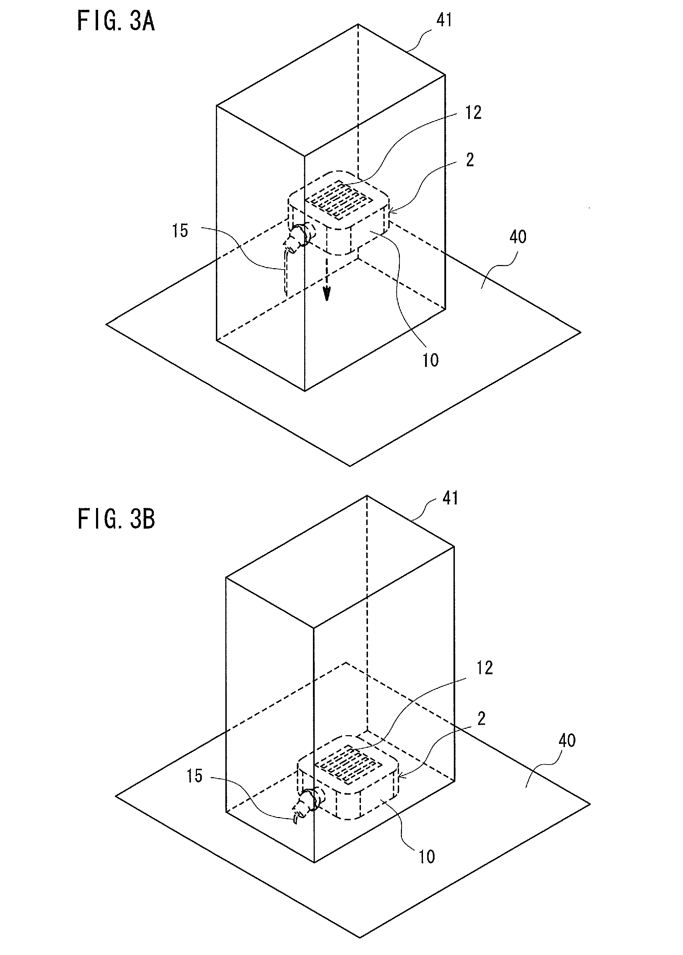

As shown in FIG. 1 and FIG. 2, the sensor 15 is formed in a long, thin belt-like shape. Also, the sensor 15 is disposed on the patient's skin, in a state where a portion 15a at the tip end is placed under the skin, using an implanting device discussed later (see FIG. 3A and B). The sensor 15 in such a state can also be said to be implanted under the skin.

Also, as shown in FIG. 2, the sensor 15 includes a substrate 18 having insulating properties and flexibility The formation material of the substrate 18 is not particularly limited. In terms of having little effect on the body, however, exemplary formation materials of the substrate 18 include thermoplastic resins such as polyethylene terephthalate (PET), polypropylene (PP) and polyethylene (PE) and thermosetting resins such as polyimide resin and epoxy resin.

Furthermore, as shown in FIG. 2, the tip of the sensor 15 can be formed into a sharp point, in order to facilitate piercing the skin. The tip is, however, not particularly limited in shape, and may be formed into a shape other than a sharp point. Also, in the present Embodiment 1, the sensor 15, being a glucose sensor, includes an electrode 16a and an electrode 16b forming a pair and a portion (enzyme reagent layer) 17 on which glucose oxidoreductase is disposed, in addition to the substrate 18.

The electrode 16a and the electrode 16b are used in order to apply voltage to the enzyme reagent layer 17. The electrode 16a and the electrode 16b are formed on the surface of the substrate 18 in the longitudinal direction of the sensor 15, and also function as wiring. The electrodes 16a and 16b can be formed, for example, by performing vapor deposition or screen printing using a non-corrosiveness metal or a conductive material such as carbon ink.

The enzyme reagent layer 17, in the example of FIG. 2, is formed by immobilizing glucose oxidoreductase on the electrode 16a. In this case, the electrode 16a functions as the working electrode. A product produced by the reaction of the glucose oxidoreductase with the glucose (substrate) in interstitial fluid or blood is detected on the electrode, and electrons generated by the reaction are passed directly or via a mediator such as a metal complex to the electrode. Accordingly, when voltage is applied between the electrodes 16a and 16b, the electrons produced by the enzyme catalyst reaction can be detected with the electrode 16a, according to the amount of reaction of the glucose in the reaction.

In the present Embodiment 1, examples of applicable glucose oxidoreductase include glucose oxidase (GOD) and glucose dehydrogenase (GDH). Furthermore, methods of immobilizing glucose oxidoreductase include various well-known methods, such as cross-linking using glutaraldehyde, for example.

Since the current value of current flowing through the electrode 16a and the electrode 16b changes according to the glucose concentration, such a configuration enables the glucose concentration to be specified by measuring this current. In the present embodiment, the current flowing through the electrode 16a and the electrode 16b is equivalent to "the signal that depends on the state of the substance."

Also, the electrode 16a and the electrode 16b provided in the sensor 15 are electrically connected to the external terminal 12 of the base 10, via wiring provided inside the variable mechanism 11 and the base 10 (not shown in FIG. 1 or FIG. 2). The external terminal 12 is thereby able to direct the signal generated by the sensor 15 to the outside.

In the present Embodiment 1, a ball joint is used as the variable mechanism 11. A shaft 11a at one end of the ball joint is attached to the portion of the sensor 15 that is not placed under the skin (portion other than the tip-end portion 15a), and a shaft 11b at the other end of ball joint is attached to the base 10. In the present Embodiment 1, the variable mechanism 11 thus enables the orientation of the sensor 15, or in other words, the orientation of the portion 15a at the tip end of the sensor 15 to be changed.

Also, in the present Embodiment 1, the control unit 3 is provided with a recessed portion 14 into which the base 10 can be fitted. The control unit 3 is attached to the base 10 by placing the control unit 3 over the base 10 disposed on the skin, and housing the base 10 within the recessed portion 14. Also, a terminal 13 for connecting to the external terminal 12 is provided in the bottom surface within the recessed portion 14, and the external terminal 12 and the terminal 13 are electrically connected when the control unit 3 is attached to the base 10.

The control unit 3 receives the signal generated by the sensor 15 via the external terminal 12 and the terminal 13 contacting therewith. Specifically, in the present Embodiment 1, the control unit 3 applies voltage to the electrode 16a and the electrode 16b of the sensor 15, and monitors the current value of current flowing through the electrode 16a and the electrode 16b. Also, the control unit 3, as arithmetic processing, generates an analog signal specifying the current value and converts the analog signal to a digital signal.

Thereafter, the control unit 3 transmits the generated digital signal to an external measurement apparatus by cable or wireless. The measurement apparatus, which is similar to a conventional apparatus, calculates the specific concentration of glucose from the received signal, and displays the calculated value on a display screen.

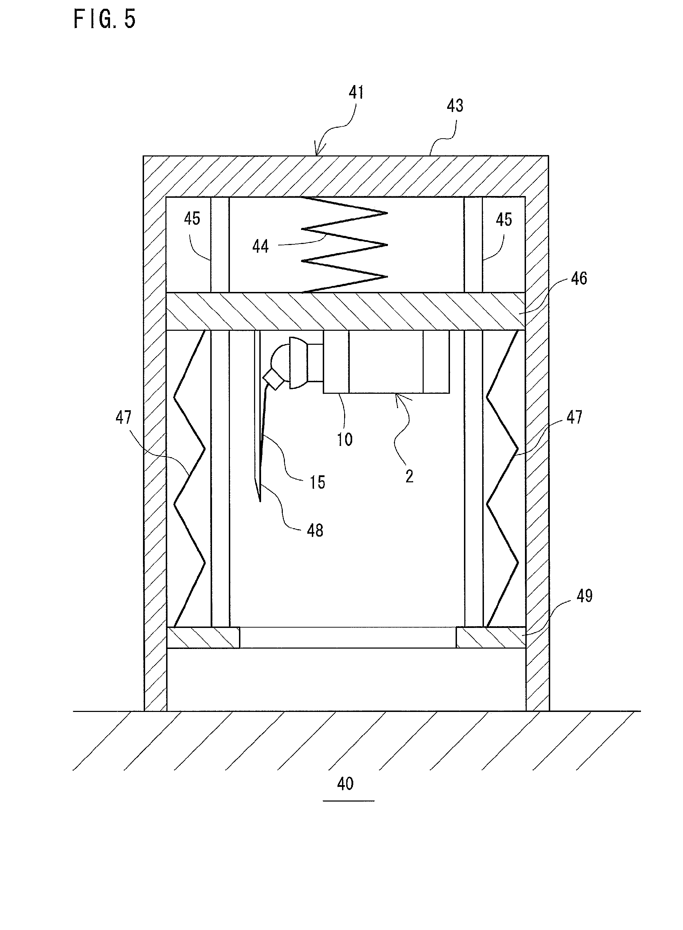

Next, the sensor placement method in Embodiment 1 of the present invention is described using FIG. 3 to FIG. 5. FIG. 3A and FIG. 3B are diagrams showing a series of steps of the sensor placement method in Embodiment 1 of the present invention. FIG. 4A and FIG. 4B are diagrams showing a series of steps of the sensor placement method in Embodiment 1 of the present invention, these steps being executed after execution of the step shown in FIG. 3B. FIG. 5 is a cross-sectional view showing an exemplary schematic configuration of the implanting device used in implementation of the sensor placement method in Embodiment 1 of the present invention.

First, as shown in FIG. 3A, the sensor unit 2 to which the sensor 15 is attached is set in an implanting device 41. The implanting device 41 is disposed on the patient's skin 40. The implanting device 41 is provided with the function of driving out the sensor unit 2 and the sensor 15 attached thereto toward the skin 40 together with a puncture needle (not shown), using an elastic body such as a spring.

Next, as shown in FIG. 3B, the sensor 15 attached to the base 10 is driven out toward the skin 40 by the implanting device 41 together with the puncture needle (not shown). At this time, the base 10 is also simultaneously sent toward the skin 40. The portion 15a at the tip end of the sensor 15 is thereby embedded in the skin 40 together with the puncture needle, and, at the same time, the base 10 is disposed on the skin 40.

The implanting device 41 also includes a mechanism for pulling back only the puncture needle after driving out the sensor 15 and the puncture needle. Therefore, the puncture needle returns to its original position after piercing the skin 40, and only the sensor 15 is placed under the skin. Note that, in the present Embodiment 1, implantation of the portion 15a of the sensor 15 in the skin 40 and disposition of the base 10 on the skin 40 favorably are performed at the same time. It is permissible, however, for there to be a time lag between the implantation and the disposition.

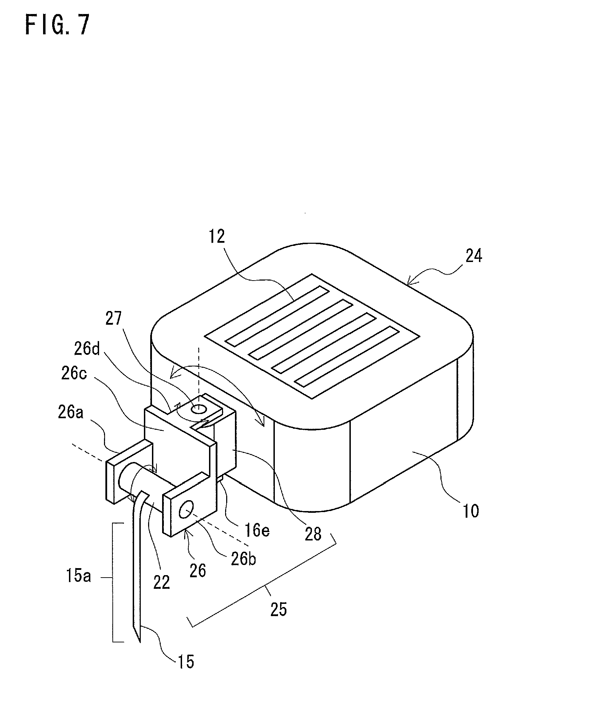

In the present Embodiment 1, the implanting device 41 need only be provided with the function of driving out the base 2, the sensor 15 and the puncture needle together, and the configuration thereof is not particularly limited. Specifically, examples of the implanting device 41 include an apparatus provided with a similar configuration to an apparatus shown in FIG. 7 to FIG. 12 of JP 2005-503243A.

Here, a specific example of the implanting device 41 is described using FIG. 5. As shown in FIG. 5, the implanting device 41 is provided with a body 43, an extrusion spring 44, a pair of guide rails 45, an extrusion member 46, a return spring 47, a puncture needle 48, and a positioning member 49.

The body 43 is formed in a cylindrical shape open at one end. The guide rails 45 are disposed in the longitudinal direction of the body. The extrusion member 46 is passed through by the guide rails 45 at two locations, and moves along the guide rails 45. Also, the projecting restriction member 49 is provided near the opening within the body 43, and the movement of the extrusion member 46 is restricted.

Also, the extrusion spring 44 is installed between the extrusion member 46 and the wall surface of the body 43 on the blocked side, and the extrusion member 46 is pushed toward the open side by the elastic force thereof. On the other hand, the return spring 47 is installed between the extrusion member 46 and the restriction member 49, and the extrusion member 46, having been pushed toward the open side, is pushed back toward its original position by the elastic force thereof.

The sensor unit 2 is disposed on the surface of the extrusion member 46 on the open side. Also, although not illustrated in FIG. 5, a holding mechanism for holding the base 10 of the sensor unit 2 is provided in the extrusion member 46. The holding mechanism is configured such that holding of the base 10 is released when the extrusion member 46 approaches furthest on the open side. Furthermore, the downwardly projecting puncture needle 48 is provided on the surface of the extrusion member 46 on the open side. The sensor 15 is in a state of being appended to the puncture needle 48.

Accordingly, if the extrusion spring 44 is contracted and released with the sensor unit 2 disposed on the extrusion member 46, the base 10 and the sensor 15 will both be pushed out forcefully toward the open side. The sensor 15 then pierces the skin 40 together with the puncture needle 48, and the base 10 contacts with the skin. Thereafter, the puncture needle 48 is pushed upward by the return spring 47 together with the extrusion member 46 and drawn out from the skin 40. If the implanting device 41 shown in FIG. 5 is used, implantation of the portion 15a of the sensor 15 in the skin 40 and disposition of the base 10 on the skin 40 are executed at the same time.

Next, the implanting device 41 is removed, as shown in FIG. 4A. The control unit 3 is then attached onto the sensor unit 2 disposed on the skin 40, as shown in FIG. 4B. The external terminal 12 provided in the base 10 and the terminal 13 of the control unit 3 (see FIG. 1) are thereby electrically connected, enabling measurement by the sensor 15. At this time, even if external force is exerted on the base 10, the external force is absorbed by the variable mechanism 11, making it extremely unlikely that the sensor 15 will move inadvertently.

As described above, in the present Embodiment 1, because movement of the sensor 15 due to external force when embedding the sensor 15 under the skin and performing measurement is suppressed, the occurrence of a situation where the function of the sensor is impaired due to expansion of the wound formed in the skin 40 is avoided. Note that situations where the function of the sensor is impaired include a situation where output of a signal from the embedded sensor 15 stops and a situation where a signal is output but measurement is difficult due a large amount of noise.

### Embodiment 2

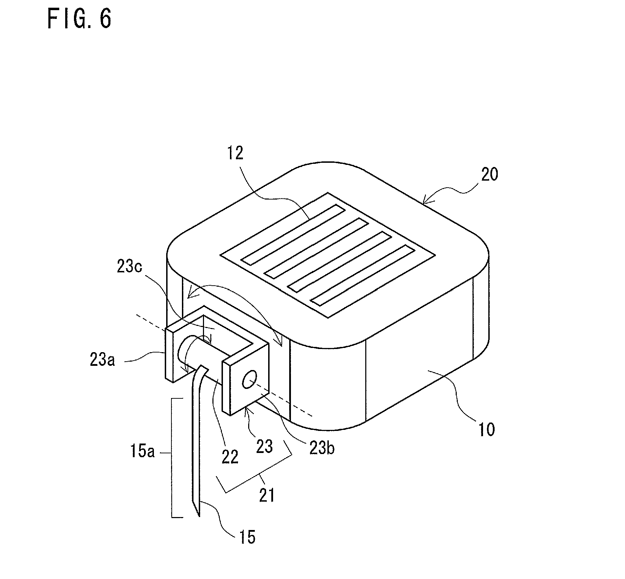

Next, a measuring apparatus and a sensor placement method in Embodiment 2 of the present invention are described, with reference to FIG. 6 and FIG. 7. Initially, a first example in the present Embodiment 2 is described. FIG. 6 is a perspective view showing a first exemplary configuration of a sensor unit in Embodiment 2 of the present invention.

As shown in FIG. 6, the sensor unit 20 in the first example of the present Embodiment 2 differs from the sensor unit 2 shown in FIG. 1 in Embodiment 1 in terms of the configuration of the variable mechanism 21. The variable mechanism 21 includes a shaft-like rotating member (rotating shaft) 22 and a holding member 23 that rotatably holds the rotating member.

The holding member 23 is provided with a plate-like portion 23c and a pair of portions 23a and 23b that project perpendicularly from the portion 23c. The holding member 23 holds both ends of the rotating member 22 with the portion 23a and the portion 23b, in a state where the rotating member 22 is rotatable. Also, while the holding member 23 is attached to the base 10 at the plate-like portion 23c, the attachment of the portion 23c to the base 10 is carried out such that the holding member 23 will be rotatable around the normal of the lateral surface of the base 10 to which the portion 23c is attached. The normal is perpendicular to the rotating member 22.

Also, the sensor 15 is attached to the rotating member 22 by the portion that is not placed under the skin (portion other than tip-end portion 15a). Accordingly, with the sensor unit 20, the orientation of the sensor 15 is changeable in two directions by the variable mechanism 21. In other words, the orientation of the sensor 15 can also be changed in the first example of the present Embodiment 2, similarly to Embodiment 1. Note that although not illustrated in FIG. 6, in the first example of the present Embodiment 2, the electrodes formed on the sensor 15 are electrically connected to the external terminal 12.

Next, a second example in the present Embodiment 2 is described. FIG. 7 is a perspective view showing a second exemplary configuration of the sensor unit in Embodiment 2 of the present invention. As shown in FIG. 7, a sensor unit 24 in the second example of the present Embodiment 2 also differs from the sensor unit 2 shown in FIG. 1 in Embodiment 1 in terms of the configuration of a variable mechanism 25.

The variable mechanism 25 includes a rotating member 22, a first holding member 26 that rotatably holds the rotating member 22, and a second holding member 28 that rotatably holds the first holding member 26. The first holding member 26 includes a plate-like portion 26c and a pair of portions 26a and 26b projecting perpendicularly from the portion 26c.

The first holding member 26, similarly to the holding member 23 of the first example shown in FIG. 6, holds both ends of the rotating member 22 with the portion 26a and the portion 26b, such that the rotating member 22 is rotatable. The sensor 15, similarly to the first example, is also attached to the rotating member 22 by the portion that is not placed under the skin (portion other than the tip-end portion 15a) in the second example. Although not illustrated in FIG. 7, electrodes formed on the sensor 15 are also electrically connected to the external terminal 12 in the second example.

Incidentally, in the second example, the first holding member 26 is also provided with a pair of portions 26d and 26e that project perpendicularly from the plate-like portion 26. The portions 26d and 26e project in opposite directions to the portions 26a and 26, and, furthermore, hold a pair of protrusions 27 that are formed in two opposing locations of the second holding member 28. Also, the protrusions 27 are held by the portions 26d and 26e such that the first holding member 26 is rotatable around an axis passing through the pair of protrusions 27 (lower protrusion is not shown). Furthermore, the portions 26d and 26e are formed such that the axis direction of the axis passing through this pair of these protrusions 27 is perpendicular to the axis direction of the rotating member 22.

The second holding member 28 is attached to the base 10, similarly to the holding member 23 of the first example. The second holding member 28 is also attached to the base 10 such that the second holding member 28 will be rotatable around the normal of the lateral surface of the base 10 to which the second holding member 28 is attached. The normal is perpendicular to both the axis direction of the rotating member 22 and the axis direction of the axis passing through the pair of protrusions 27.

With the sensor unit 24, the variable mechanism 25 thus includes three axes of rotation, and the orientation of the sensor 15 is changeable in three directions. According to the second example, the orientation of the sensor 15 can be changed with more degrees of freedom, compared to the first example.

Also, the control unit 3 shown in FIG. 1 in Embodiment 1 can be attached to either of the sensor units 20 and 24 in the present Embodiment 2. The measuring apparatus in the present Embodiment 2 can be constituted by attaching the control unit 3 to the sensor unit 20 or 24. Furthermore, the sensor placement method in the present Embodiment 2 is implemented according to the steps shown in FIG. 3A to FIG. 4B in Embodiment 1.

As described above, movement of the sensor 15 due to external force when embedding the sensor 15 under the skin and performing measurement is also suppressed in the present Embodiment 2, similarly to Embodiment 1. The occurrence of a situation where the function of the sensor 15 is impaired due to expansion of the wound formed in the skin 40 is also avoided in the case where the present Embodiment 2 is used.

### Embodiment 3

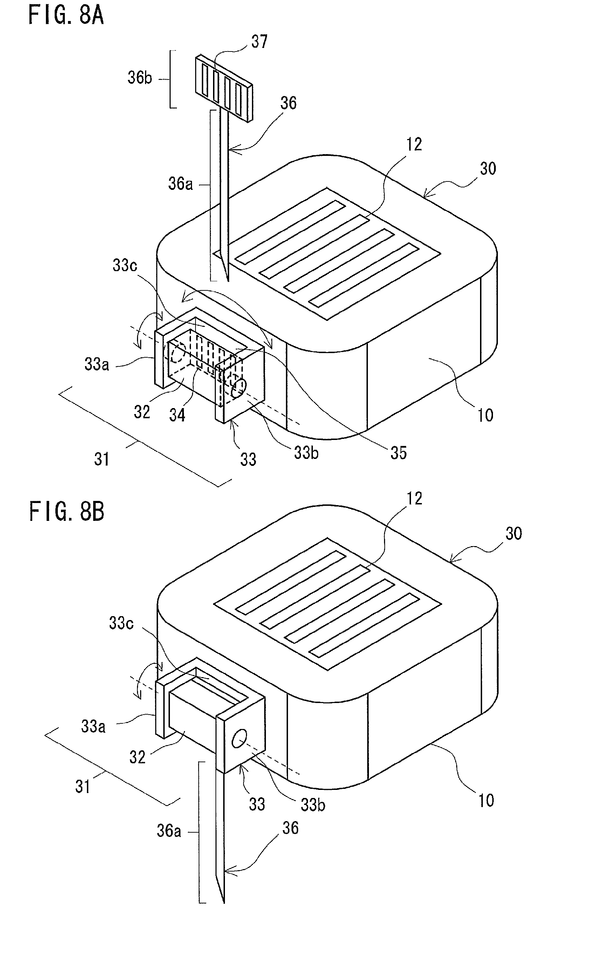

Next, a measuring apparatus, a sensor unit and a sensor placement method that uses the measuring apparatus and the sensor unit in Embodiment 3 of the present invention are described, with reference to FIG. 8 to FIG. 10. Initially, the configuration of a sensor unit 30 in the present Embodiment 3 is described using FIG. 8. FIG. 8 includes perspective views showing the configuration of the sensor unit in Embodiment 3 of the present invention, FIG. 8A showing a state where the sensor is removed, and FIG. 8B showing a state where the sensor is attached.

As shown in FIG. 8A and FIG. 8B, the sensor unit 30 is provided with a variable mechanism 31. The variable mechanism 31 includes a rotating member 32 and a holding member 33 that rotatably holds the rotating member 32, similarly to the variable mechanism 21 shown in the first example of Embodiment 2 (see FIG. 6).

The holding member 33 is provided with a plate-like portion 33c and a pair of portions 33a and 33b that project perpendicularly from the plate-like portion 33c, similarly to the holding member 23 (see FIG. 7). The holding member 33 holds both ends of the rotating member 32 with the portion 33a and the portion 33b, in a state where the rotating member 32 is rotatable. Furthermore, the holding member 33, similarly to the holding member 23 (see FIG. 7), is attached to the base 10 at the plate-like portion 33c, so as to be rotatable around the normal of the lateral surface of the base 10.

In the present Embodiment 3, the variable mechanism 31, although thus provided with a similar configuration to the variable mechanism 21 shown in the first example of Embodiment 2 (see FIG. 6), differs from the first example of Embodiment 2 in terms of the holding of the sensor 36 by the variable mechanism 31. This is described hereinafter.

In the present Embodiment 3, as shown to FIG. 8A, the sensor 36 can be removed from the variable mechanism 31. The sensor 36 includes a portion (tip-end portion) 36a that is embedded under the skin, and a portion (base-end portion) 36b that is held by the variable mechanism 31. Also, the sensor 36, similarly to the sensor 15 shown in FIG. 2, includes a substrate, an enzyme reagent layer formed thereon, and a pair of electrodes likewise formed thereon. Furthermore, a connection terminal 37 electrically connected to the electrodes (see FIG. 2) formed on the sensor 36 is provided at the base-end portion 36b.

Also, in the variable mechanism 31, a terminal 34 connectible to the connection terminal 37 is provided on the portion 33c side of the rotating member 32. Furthermore, although not illustrated in FIG. 8A or B, the terminal 34 and the external terminal 12 provided in the base 10 are electrically connected.

As shown in FIG. 8B, the sensor 36, at the time of usage, is inserted into a slit 35 formed with the rotating member 32 and the portion 33c, and is thereby held by the variable mechanism 31. At this time, the connection terminal 37 of the sensor 36 and the terminal 34 provided in the rotating member 32 are electrically connected, resulting in the electrodes formed on the sensor 36 (see FIG. 2) being electrically connected to the external terminal 12.

According to the present Embodiment 3, the sensor 36 can thus be easily removed from the variable mechanism 31. In the present Embodiment 3, the orientation of the sensor 36 is also changeable in two directions by the variable mechanism 31, similarly to the first example of Embodiment 2. Furthermore, the control unit 3 shown in FIG. 1 in Embodiment 1 can also be attached to the sensor unit 30 in the present Embodiment 3. The measuring apparatus in the present Embodiment 3 can be constituted by attaching the control unit 3 to the sensor unit 30.

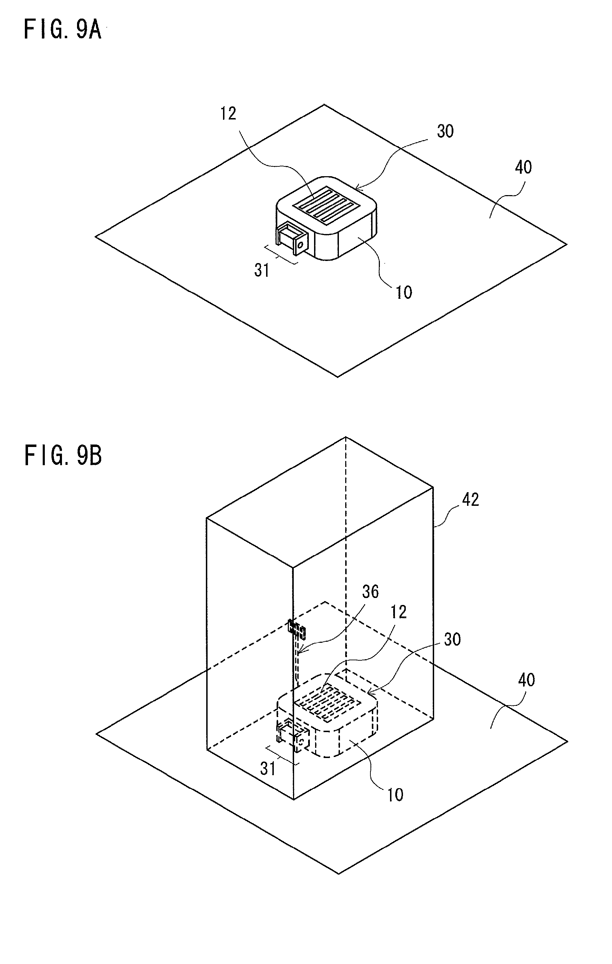

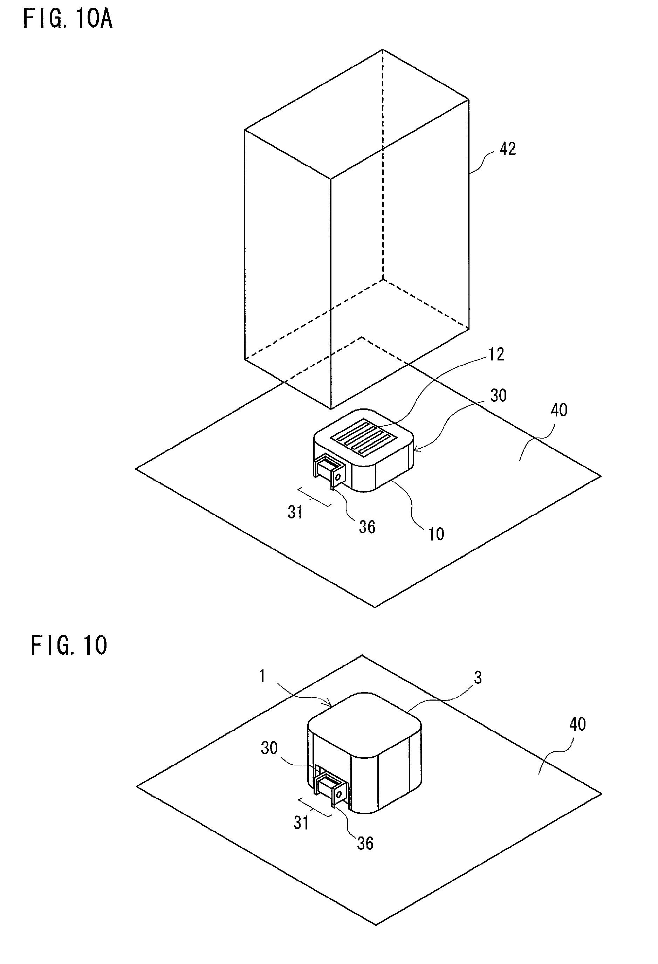

Next, the sensor placement method in Embodiment 3 of the present invention is described using FIG. 9 and FIG. 10. FIG. 9A and FIG. 9B are diagrams showing a series of steps of the sensor placement method in Embodiment 2 of the present invention. FIG. 10A and FIG. 10B are diagrams showing a series of steps of the sensor placement method in Embodiment 2 of the present invention, these steps being executed after execution of the step shown in FIG. 9B.

First, the sensor unit 30 to which the sensor 36 is not attached is disposed on the patient's skin 40, as shown in FIG. 9A. Next, an implanting device 42 in which the sensor 36 has been set is disposed over the sensor unit 30, as shown in FIG. 9B.

The implanting device 42 is provided with the function of driving out the sensor 36 toward the skin 40 together with a puncture needle (not shown), using an elastic body such as a spring. Also, the implanting device 42 is disposed such that the sensor 36 is inserted in the slit 35 (see FIG. 8A) formed between the rotating member 32 and the portion 33c, after being driven in.

In the present Embodiment 3, the implanting device 42, unlike the implanting device 41, need only be provided with the function of driving out only the sensor 36 and the puncture needle toward the skin 40, and the configuration thereof is not particularly limited. Examples of the implanting device 42 include an apparatus provided with a similar configuration to an apparatus shown in FIG. 6 to FIG. 8 of US Patent No. 7310544.

Next, as shown in FIG. 10A, the sensor 36 is driven out toward the skin 40 by the implanting device 42 together with the puncture needle (not shown), and the portion 36a at the tip end of the sensor 36 is implanted in the skin 40 together with the puncture needle. Also, the implanting device 42 is provided with a mechanism for pulling back only the puncture needle after driving out the sensor 36 and the puncture needle. Therefore, the puncture needle returns to its original position after having pierced the skin 40, and only the sensor 36 is placed under the skin.

Also, the connection terminal 37 of the sensor 36 and the terminal 34 provided in the rotating member 32 are electrically connected at the same time as the implantation of the sensor 36 shown in FIG. 10A. The electrodes formed on the sensor 36 (see FIG. 2) and the external terminal 12 are thereby electrically connected. The implanting device 42 is removed once the sensor 36 has been implanted. Note that, in the present Embodiment 3, the connection terminal 37 and the terminal 34 favorably are electrically connected at the same time as the implantation of the sensor 36 in the skin 40. It is permissible, however, for there to be a time lag between the implantation and the electrical connection.

The control unit 3 is then attached onto the sensor unit 30 disposed on the skin 40, as shown in FIG. 10B. The external terminal 12 provided in the base 10 and the terminal 13 of the control unit 3 (see FIG. 1) are thereby electrically connected, enabling measurement by the sensor 36. Also, at this time, even if external force is exerted on the base 10, the external force is absorbed by the variable mechanism 31, making it extremely unlikely that the sensor 36 will move inadvertently.

As described above, because movement of the sensor 36 due to external force when embedding the sensor 36 under the skin and performing measurement is also suppressed in the case where the present Embodiment 3 is used, the occurrence of a situation where the function of the sensor 36 is impaired due to expansion of the wound formed in the skin 40 is avoided.

Also, although not illustrated in the above-mentioned Embodiments 1 to 3, in the present invention the variable mechanism preferably is provided with a function of locking the position and orientation of the sensor. The possibility of the sensor moving inadvertently due to external force exerted on the base after the sensor has been embedded and the control unit has been attached decreases, and locking the position and orientation of the sensor in fact increases the possibility of being able to avoid a situation where the sensor moves due an external impact.

Furthermore, although the sensor 15 (or 36) is connected to the terminal 13 of the control unit 3 (see FIG. 1) via the external terminal 12 provided in the sensor unit 2 (20 or 30) in the above-mentioned Embodiments 1 to 3, the present invention is not limited to this mode. The present invention may, for example, adopt a mode in which the connection terminal 37 of the sensor 36 (FIG. 8A) is electrically connected directly to the terminal 13 of the control unit 3. In this case, the control unit is able to directly receive the signal from the sensor 36.

### Embodiment 4

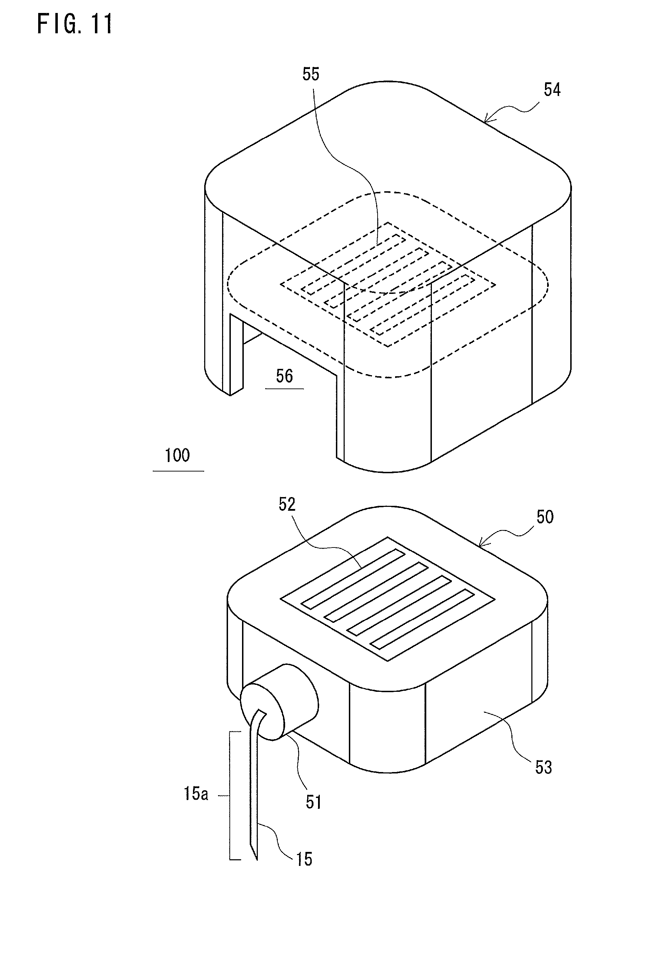

Next, a measuring apparatus and a sensor placement method in Embodiment 4 of the present invention are described, with reference to FIG. 11 to FIG. 13. Initially, the configuration of the measuring apparatus in the present Embodiment 4 is described using FIG. 11. FIG. 11 is a perspective view showing the configuration of the measuring apparatus in Embodiment 4 of the present invention.

A measuring apparatus 100 in the present Embodiment 4 shown in FIG. 11 is an apparatus that measures numerical information relating to a substance contained in a body fluid within the body, similarly to the measuring apparatuses shown in Embodiments 1 to 3. As shown in FIG. 11, the measuring apparatus 100 is provided with a sensor unit 50 and a control unit 54. Note that examples of body fluid within the body include interstitial fluid, blood and plasma. Furthermore, in the present specification, "within the body" includes "under the skin" indicating below the skin surface.

The sensor unit 50 is provided with a base 53, an external terminal 52, a sensor 15, and a sensor holding member 51. Of these, the sensor 15 is similar to the sensor 15 shown in FIG. 2 in Embodiment 1, and is partially placed within the patient's body, or specifically, under the patient's skin, in order to execute CGM (see FIG. 2). The sensor unit 50 also functions as a sensor placement apparatus for placing the sensor 15. Also, the sensor 15 generates a signal according to the state of the substance contained in the body fluid within the body.

The base 53, similarly to the base 10 shown in FIG. 1, is disposed on the skin of the patient who is being measured and holds the sensor 15. The sensor holding member 51 is attached to the base 53, and interposes between the base 53 and the sensor 15. Also, the external terminal 52, similarly to the external terminal 12 shown in FIG. 1, is provided in the base 53 and electrically connected to the sensor 15. The external terminal 52 is able to direct the signal generated by the sensor 15 to the outside.

The control unit 54, similarly to the control unit 3 shown in FIG. 1, receives the signal generated by the sensor 15 via the external terminal 52, and executes processing based on the received signal. The signal generated by the sensor 15 is also sent to the control unit 54 via the external terminal 52 provided in the base 53 in the present Embodiment 4. Also, the control unit 54 is formed so as to be attachable to the base 53.

In the present Embodiment 4, the sensor 15 is thus connected to the control unit 54 via the external terminal 52 provided in the base 53. For this reason, the load placed on the portion of the sensor 15 inserted under the skin when connecting the sensor 15 and the control unit 54 is reduced. A poor connection between the sensor 15 and the control unit 54 is also unlikely to occur.

Here, the configurations of the measuring apparatus 100 in the present Embodiment 4 and the sensor unit 50 and the control unit 54 constituting the measuring apparatus 100 are more specifically described.

In the present Embodiment 4, the substance that is measured is glucose in interstitial fluid, similarly to Embodiment 1, and the numerical information relating to the substance is the concentration of glucose. The sensor 15 generates a signal according to the state (concentration) of glucose in interstitial fluid. In the following, an example is described in which the numerical information relating to the substance is the concentration of glucose, and the sensor 15 is a glucose sensor. Note that similarly in the present Embodiment 4, the substance that is measured may be a substance other than glucose, and may be a substance in blood. Also, the numerical information may be information other than concentration.

In the present Embodiment 4, the sensor 15 is similarly provided with the configuration shown in FIG. 2 in Embodiment 1. In the present Embodiment 4, the sensor 15 is, however, partially inserted inside the sensor holding member 51, and held by the sensor holding member 51. The electrodes 16a and 16b of the sensor 15 are electrically connected to the external terminal 52 via the inside of the sensor holding member 51.

Such a configuration enables the sensor 15 to continuously output a signal that depends on the state of glucose in interstitial fluid, and allow the measuring apparatus 100 to function as a monitoring apparatus capable of continuously monitoring the concentration of glucose. In this case, the measuring apparatus 100 is able to execute the above-mentioned CGM.

Furthermore, in the present Embodiment 4, the control unit 54 is also provided with a recessed portion 56 into which the base 10 can be fitted, similarly to the control unit 3 shown in FIG. 1. Attachment of the control unit 54 to the base 53 is also carried out by placing the control unit 54 over the base 53 disposed on the skin, and housing the base 53 within the recessed portion 56. Also, a terminal 55 for connecting to the external terminal 52 is provided in the bottom surface within the recessed portion 56, and the external terminal 52 and the terminal 55 are electrically connected when the control unit 54 is attached to the base 53.

Also, the control unit 54, similarly to the control unit 3, receives the signal generated by the sensor 15, via the external terminal 52 and the terminal 55 contacting therewith. Specifically, in the present Embodiment 4, the control unit 54 similarly applies voltage to the electrode 16a and the electrode 16b of the sensor 15 (see FIG. 2) and monitors the current value of current flowing through the electrode 16a and the electrode 16b. Also, the control unit 54, as arithmetic processing, generates an analog signal specifying the current value and converts the analog signal to a digital signal.

Thereafter, the control unit 3 transmits the generated digital signal to an external measurement apparatus by cable or wireless. The measurement apparatus, which is similar to a conventional apparatus, calculates the specific concentration of glucose from the received signal, and displays the calculated value on a display screen.

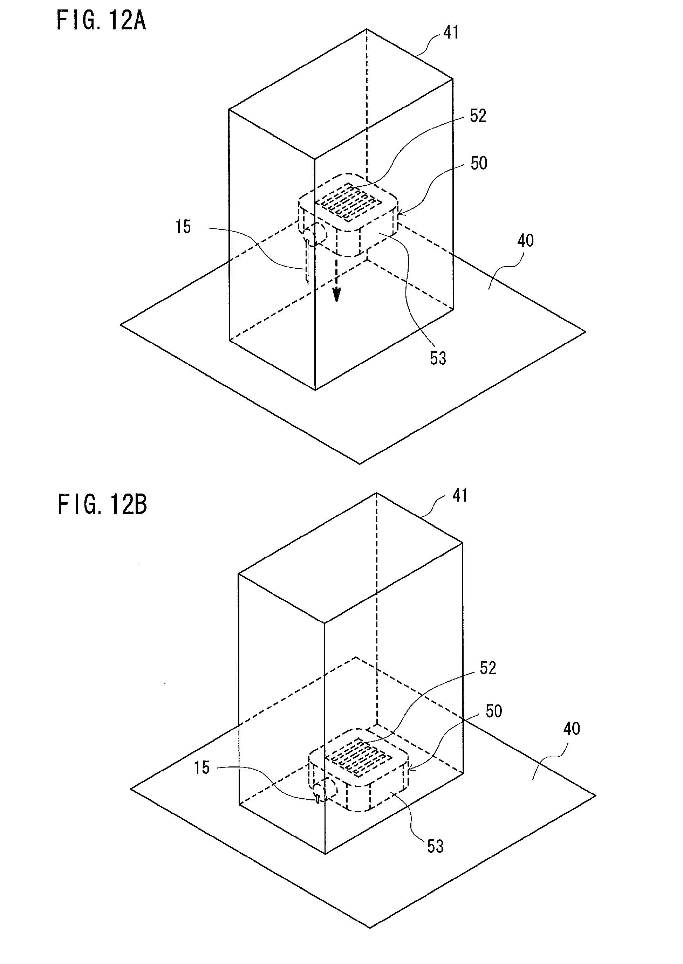

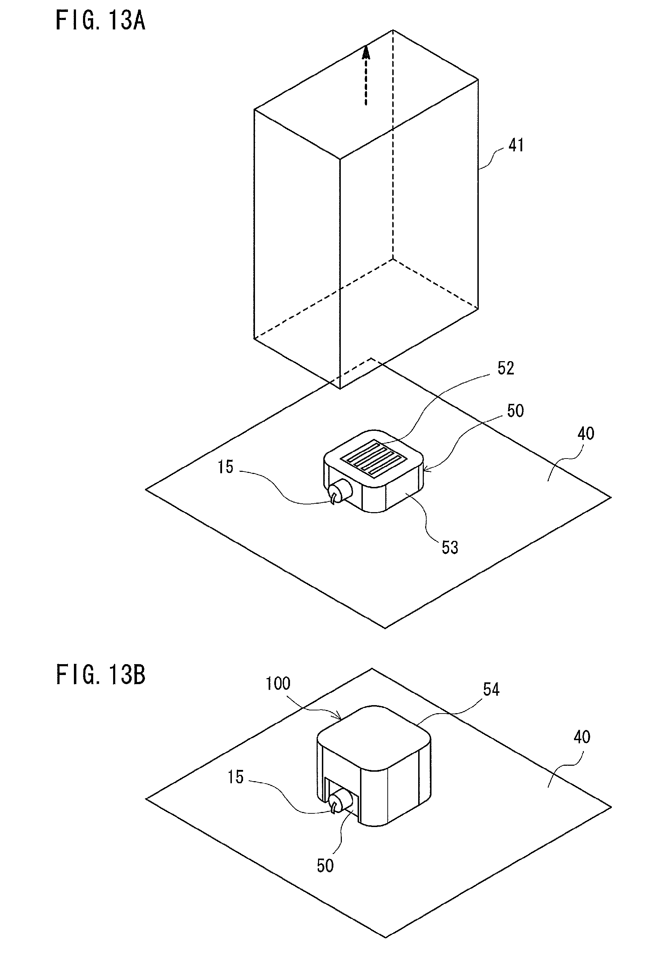

Next, the sensor placement method in Embodiment 4 of the present invention is described using FIG. 12 and FIG. 13. FIG. 12A and FIG. 12B are diagrams showing a series of steps of the sensor placement method in Embodiment 4 of the present invention. FIG. 13A and FIG. 13B are diagrams showing a series of steps of the sensor placement method in Embodiment 4 of the present invention, these steps being executed after execution of the step shown in FIG. 12B.

First, as shown in FIG. 12A, the sensor unit 50 to which the sensor 15 is attached is set in an implanting device 41. The implanting device 41, which is similar to the implanting device 41 shown in FIG. 5 in Embodiment 1, is disposed on the patient's skin 40.

Next, as shown in FIG. 12B, the sensor 15 attached to the base 50 is driven out toward the skin 40 by the implanting device 41 together with a puncture needle (see FIG. 5). At this time, the base 50 is also simultaneously sent toward the skin 40. The portion 15a at the tip end of the sensor 15 is thereby implanted in the skin 40 together with the puncture needle, and, at the same time, the base 50 is disposed on the skin 40.

Next, the implanting device 41 is removed, as shown in FIG. 13A. The control unit 54 is then attached onto the sensor unit 50 disposed on the skin 40, as shown in FIG. 13B. The external terminal 52 provided in the base 53 and the terminal 55 of the control unit 54 (see FIG. 1) are thereby electrically connected, enabling measurement by the sensor 15.

As described above, in the present Embodiment 4, the external terminal 52 is provided in the base 53, allowing the load placed on the portion of the sensor 15 inserted under the skin when connecting the sensor 15 and the control unit 54 to be reduced. As a result, the occurrence of a situation where the function of the sensor 15 is impaired due to expansion of the wound formed in the skin 40 is avoided. A poor connection between the sensor 15 and the control unit 54 is also unlikely to occur.

### Embodiment 5

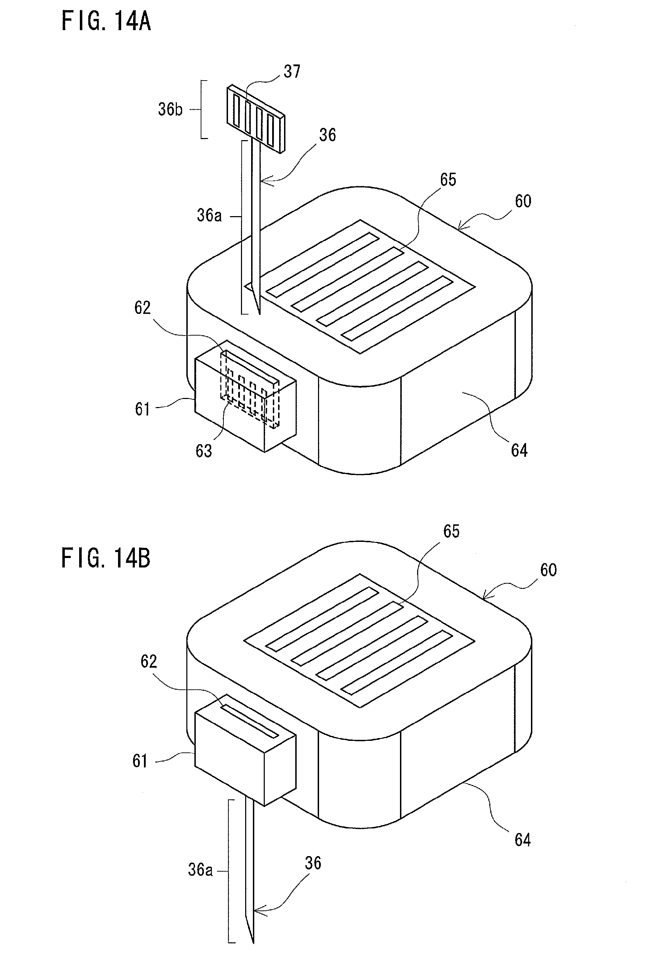

Next, a measuring apparatus and a sensor placement method in Embodiment 5 of the present invention are described, with reference to FIG. 14 to FIG. 16. Initially, the configuration of a sensor unit 30 in the present Embodiment 5 is described using FIG. 14. FIG. 14 includes perspective views showing the configuration of the sensor unit in Embodiment 5 of the present invention, FIG. 14A showing a state where the sensor is removed, and FIG. 14B showing a state where the sensor is attached.

As shown in FIG. 14A and FIG. 14B, the sensor unit 60 is provided with a base 64, an external terminal 65, a sensor 36, and a sensor holding member 61. Of these, the base 64 and the external terminal 65 are constituted similarly to the base 53 and the external terminal 52 shown in FIG. 11 in Embodiment 3.

Also, the sensor 36 is similar to the sensor 36 shown in FIG. 8A and FIG. 8B in Embodiment 3. The sensor 36 is provided with a portion (tip-end portion) 36a that is embedded under the skin, and a portion (base-end portion) 36b that is held by the sensor holding member 61. Also, a connection terminal 37 electrically connected to electrodes formed on the sensor 36 (see FIG. 2) is provided at the base-end portion 36b of the sensor 36.

The sensor holding member 61 is attached to the base 64, and interposes between the base 64 and the sensor 36. The sensor holding portion 61 is provided with a slit 62. The slit 62 is formed such that the sensor 36 can be inserted therein, and a terminal 63 connectible to the connection terminal 37 of the sensor 36 is provided on an inner wall surface thereof. Also, although not illustrated in FIG. 14A or FIG. 14B, the terminal 63 and the external terminal 65 provided in the base 64 are electrically connected.

Accordingly, when the sensor 36 has been inserted in the slit 35 at the time of usage, as shown in FIG. 14B, the connection terminal 37 of the sensor 36 and the terminal 63 provided in the sensor holding member 61 are electrically connected, resulting in the electrodes formed on the sensor 36 (see FIG. 2) and the external terminal 65 being electrically connected.

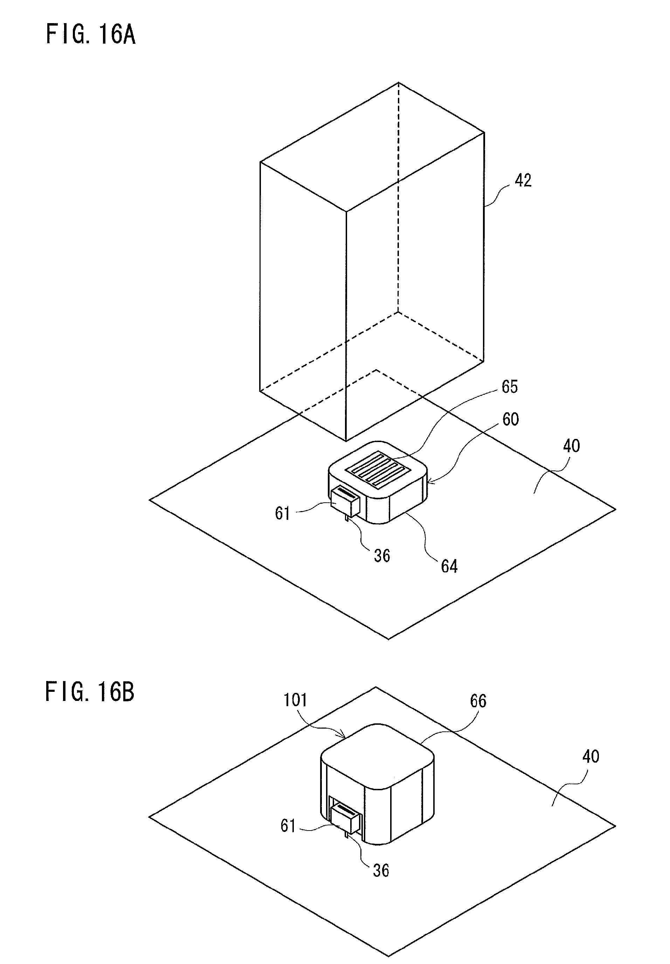

In this way, in the present Embodiment 5, unlike Embodiment 4, the sensor 36 can be easily removed from the sensor holding member 61. A control unit 66 (see FIG. 16B) similar to the control unit 3 shown in FIG. 1 in Embodiment 1 can also be attached to the sensor unit 60 in the present Embodiment 5. The measuring apparatus in the present Embodiment 5 is constituted by attaching the control unit 66 to the sensor unit 60.

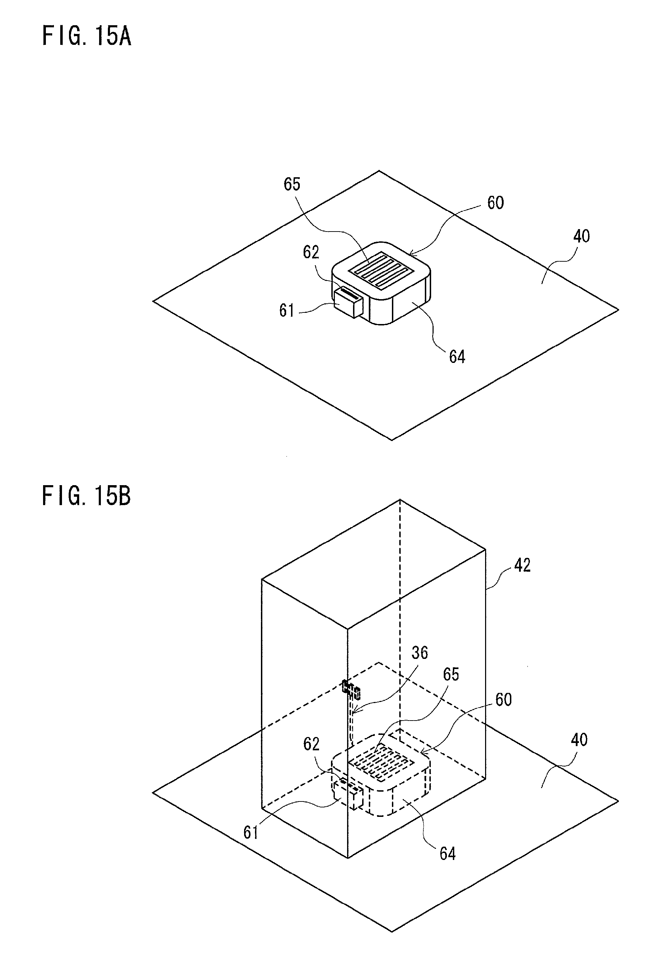

Next, the sensor placement method in Embodiment 5 of the present invention is described using FIG. 15 and FIG. 16. FIG. 15A and FIG. 15B are diagrams showing a series of steps of the sensor placement method in Embodiment 5 of the present invention. FIG. 16A and FIG. 16B are diagrams showing a series of steps of the sensor placement method in Embodiment 5 of the present invention, these steps being executed after execution of the step shown in FIG. 15B.

First, as shown in FIG. 15A, the sensor unit 60 to which the sensor 36 is not attached is disposed on the patient's skin 40. Next, as shown in FIG. 15B, an implanting device 42 in which the sensor 36 has been set is disposed over the sensor unit 60.

The implanting device 42, which is similar to the implanting device shown in FIG. 9A in Embodiment 3, is provided with the function of driving out the sensor 36 toward the skin 40 together with a puncture needle (not shown), using an elastic body such as a spring. Also, the implanting device 42 is disposed such that the sensor 36 is inserted into the slit 62 of the sensor holding member 61 (see FIG. 14A) after being driven in.

Note that the configuration of the implanting device 42 is also not particularly limited in the present Embodiment 5, and examples of the implanting device 42 include an apparatus provided with a similar configuration to an apparatus shown in FIG. 6 to FIG. 8 of US Patent No. 7310544.

Next, as shown in FIG. 16A, the sensor 36 is driven out toward the skin 40 by the implanting device 42 together with the puncture needle (not shown), and the portion 36a at the tip end of the sensor 36 is implanted in the skin 40 together with the puncture needle.

Also, the connection terminal 37 of the sensor 36 and the terminal 63 provided in the sensor holding member 61 are electrically connected at the same time as the implantation of the sensor 36 shown in FIG. 16A. The electrodes formed on the sensor 36 (see FIG. 2) and the external terminal 65 are thereby electrically connected. The implanting device 42 is removed once the sensor 36 has been implanted. Note that, in the present Embodiment 5, the connection terminal 37 and the terminal 63 favorably are electrically connected at the same time as the implantation of the sensor 36 in the skin 40. It is permissible, however, for there to be a time lag between the implantation and the electrical connection.

The control unit 66 is then attached onto the sensor unit 60 disposed on the skin 40, as shown in FIG. 16B. The external terminal 65 provided in the base 64 and the terminal of the control unit 66 (see FIG. 1) are thereby electrically connected, enabling measurement by the sensor 36.

As described above, in the present Embodiment 5, the external terminal 65 is similarly provided in the base 64, allowing the load placed on the portion of the sensor 36 inserted under the skin when connecting the sensor 36 and the control unit 66 to be reduced. As a result, the occurrence of a situation where the function of the sensor 36 is impaired due to expansion of the wound formed on the skin 40 is avoided. A poor connection between the sensor 36 and the control unit 66 is also unlikely to occur.

While some or all of the above-mentioned embodiments can be represented by the following supplementary notes 1 to 40, implementation of the present invention is not limited to the following description.

### (Supplementary note 1)

A measuring apparatus for measuring numerical information relating to a substance contained in a body fluid within a body, comprising a sensor unit and a control unit,
wherein the sensor unit includes:
a sensor that generates a signal according to a state of the substance;
a base that holds the sensor; and
a variable mechanism that is attached to the base and enables at least one of a position and an orientation of the sensor to be changed, and
the control unit is formed so as to be attachable to the base, and executes processing after receiving the signal generated by the sensor.

### (Supplementary note 2)

The measuring apparatus according to supplementary note 1, wherein the variable mechanism includes a ball joint, and
a shaft at one end of the ball joint is attached to the sensor and a shaft at the other end of the ball joint is attached to the base.

### (Supplementary note 3)

The measuring apparatus according to supplementary note 1,
wherein the variable mechanism includes a rotating member that is held in a rotatable state, and
the rotating member is attached to the sensor.

### (Supplementary note 4)

The measuring apparatus according to any of supplementary notes 1 to 3,
wherein the sensor unit further includes an external terminal that is provided in the base and directs the signal generated by the sensor to the outside, and
the control unit includes a terminal that contacts with the external terminal included in the base, when the control unit is attached to the base, and receives the signal generated by the sensor via the external terminal and the terminal contacting therewith.

### (Supplementary note 5)

The measuring apparatus according to any of supplementary notes 1 to 3,
wherein the sensor includes a connection terminal for connecting to the outside, and
the control unit includes a terminal that contacts with the connection terminal included in the sensor, when the control unit is attached to the base, and receives the signal generated by the sensor via the terminal contacting with the connection terminal.

### (Supplementary note 6)

A sensor unit comprising:
a sensor that generates a signal according to a state of a substance contained in a body fluid within a body;
a base that holds the sensor; and
a variable mechanism that is attached to the base and enables at least one of a position and an orientation of the sensor to be changed.

### (Supplementary note 7)

The sensor unit according to supplementary note 6,
wherein the variable mechanism includes a ball joint, and
a shaft at one end of the ball joint is attached to the sensor and a shaft at the other end of the ball joint is attached to the base.

### (Supplementary note 8)

The sensor unit according to supplementary note 6,
wherein the variable mechanism includes a rotating member that is held in a rotatable state, and
the rotating member is attached to the sensor.

### (Supplementary note 9)

A sensor placement apparatus comprising:
a sensor that generates a signal according to a state of a substance contained in a body fluid in a body;
a base that holds the sensor; and
a variable mechanism that is attached to the base and enables at least one of a position and an orientation of the sensor to be changed.

### (Supplementary note 10)

The sensor placement apparatus according to supplementary note 9,
wherein the variable mechanism includes a ball joint, and
a shaft at one end of the ball joint is attached to the sensor and a shaft at the other end of the ball joint is attached to the base.

### (Supplementary note 11)

The sensor placement apparatus according to supplementary note 9,
wherein the variable mechanism includes a rotating member that is held in a rotatable state, and
the rotating member is attached to the sensor.

### (Supplementary note 12)

A sensor placement method for placing a sensor within a body, the sensor generating a signal according to a state of a substance contained in a body fluid within the body, comprising the steps of:
(a) disposing a base on skin in a state where the sensor is held by the base via a variable mechanism that is attached to the base and enables at least one of a position and an orientation of the sensor to be changed, and partially implanting the sensor within the body;
(b) attaching a control unit to the base, the control unit executing processing after receiving the signal generated by the sensor.

### (Supplementary note 13)

The sensor placement method according to supplementary note 12,
wherein the variable mechanism includes a ball joint, and
a shaft at one end of the ball joint is attached to the sensor and a shaft at the other end of the ball joint is attached to the base.

### (Supplementary note 14)

The sensor placement method according to supplementary note 12, wherein the variable mechanism includes a rotating member that is held in a rotatable state, and
the rotating member is attached to the sensor.

### (Supplementary note 15)

The sensor placement method according to any of supplementary notes 12 to 14,
wherein an external terminal that directs the signal generated by the sensor to the outside is provided in the base,
the control unit includes a terminal that contacts with the external terminal included in the base, and
the step (b) comprises connecting the external terminal provided in the base and the terminal included in the control unit.

### (Supplementary note 16)

The sensor placement method according to any of supplementary notes 12 to 14,
wherein the sensor includes a connection terminal for connecting to the outside,
the control unit includes a terminal that contacts with the connection terminal included in the sensor, and
the step (b) comprises connecting the connection terminal included in the sensor and the terminal included in the control unit.

### (Supplementary note 17)

A sensor placement method for placing a sensor within a body, the sensor generating a signal according to a state of a substance contained in a body fluid within the body, comprising the steps of:
(a) disposing a base on skin, the base having attached thereto a variable mechanism that enables at least one of a position and an orientation of the sensor to be changed;
(b) partially implanting the sensor within the body, and causing the sensor to be held by the base via the variable mechanism; and
(c) attaching a control unit to the base, the control unit executing processing after receiving the signal generated by the sensor.

### (Supplementary note 18)

The sensor placement method according to supplementary note 17,
wherein the variable mechanism includes a ball joint, and
a shaft at one end of the ball joint is attached to the sensor and a shaft at the other end of the ball joint is attached to the base.

### (Supplementary note 19)

The sensor placement method according to supplementary note 17,
wherein the variable mechanism includes a rotating member that is held in a rotatable state, and
the rotating member is attached to the sensor.

### (Supplementary note 20)

The sensor placement method according to any of supplementary notes 17 to 19,
wherein an external terminal that directs the signal generated by the sensor to the outside is provided in the base,
the control unit includes a terminal that contacts with the external terminal included in the base, and
the step (c) comprises connecting the external terminal provided in the base and the terminal included in the control unit.

### (Supplementary note 21)

The sensor placement method according to any of supplementary notes 17 to 19,
wherein the sensor includes a connection terminal for connecting to the outside,
the control unit includes a terminal that contacts with the connection terminal included in the sensor, and
the step (c) comprises connecting the connection terminal included in the sensor and the terminal included in the control unit.

### (Supplementary note 22)

A sensor placement method for placing a sensor that generates a signal according to a state of a substance contained in a body fluid within a body, comprising the steps of
(a) disposing a base on skin in a state where the sensor is held by the base, and partially implanting the sensor under the skin; and
(b) attaching a control unit to the base, the control unit executing processing after receiving the signal generated by the sensor.

### (Supplementary note 23)

The sensor placement method according to supplementary note 22, wherein a variable mechanism that enables at least one of a position and an orientation of the sensor to be changed is attached to the base.

### (Supplementary note 24)

The sensor placement method according to supplementary note 23,
wherein the variable mechanism includes a ball joint, and
a shaft at one end of the ball joint is attached to the sensor and a shaft at the other end of the ball joint is attached to the base.

### (Supplementary note 25)

The sensor placement method according to supplementary note 23,
wherein the variable mechanism includes a rotating member that is held in a rotatable state, and
the rotating member is attached to the sensor.

### (Supplementary note 26)

The sensor placement method according to any of supplementary notes 23 to 25,
wherein an external terminal that directs the signal generated by the sensor to the outside is provided in the base,
the control unit includes a terminal that contacts with the external terminal included in the base, and
the step (b) comprises connecting the external terminal provided in the base and the terminal included in the control unit.

### (Supplementary note 27)

The sensor placement method according to any of supplementary notes 23 to 25,
wherein the sensor includes a connection terminal for connecting to the outside,
the control unit includes a terminal that contacts with the connection terminal included in the sensor, and
the step (b) comprises connecting the connection terminal included in the sensor and the terminal included in the control unit.

### (Supplementary note 28)

A measuring apparatus for measuring numerical information relating to a substance contained in a body fluid within a body, comprising a sensor unit and a control unit,
wherein the sensor unit includes:
a sensor that generates a signal according to a state of the substance;
a base that holds the sensor; and
an external terminal that is provided in the base and directs the signal generated by the sensor to the outside, and
the control unit is formed so as to attachable to the base, and executes processing after receiving the signal generated by the sensor via the external terminal.

### (Supplementary note 29)

The measuring apparatus according to supplementary note 28, wherein the sensor unit further includes a variable mechanism that is attached to the base and enables at least one of a position and an orientation of the sensor to be changed.

### (Supplementary note 30)

The measuring apparatus according to supplementary note 28 or 29, wherein the control unit includes a terminal that contacts with the external terminal included in the base, when attached to the base, and receives the signal generated by the sensor via the external terminal and the terminal contacting therewith.

### (Supplementary note 31)

A sensor unit comprising:
a sensor that generates a signal according to a state of a substance contained in a body fluid within a body;
a base that holds the sensor; and
an external terminal that is provided in the base and directs the signal generated by the sensor to the outside.

### (Supplementary note 32)

The sensor unit according to supplementary note 31 further comprising a variable mechanism that is attached to the base and enables at least one of a position and an orientation of the sensor to be changed.

### (Supplementary note 33)

A sensor placement apparatus comprising:
a sensor that generates a signal according to a state of a substance contained in a body fluid within a body;
a base that holds the sensor; and
an external terminal that is provided in the base and directs the signal generated by the sensor to the outside.

### (Supplementary note 34)

The sensor placement apparatus according to supplementary note 33 further comprising a variable mechanism that is attached to the base and enables at least one of a position and an orientation of the sensor to be changed.

### (Supplementary note 35)

A sensor placement method for placing a sensor that generates a signal according to a state of a substance contained in a body fluid within a body, comprising the steps of
(a) disposing a base on skin in a state where the sensor is held by the base which is provided with an external terminal that directs the signal generated by the sensor to the outside, and partially implanting the sensor within the body; and
(b) attaching a control unit to the base, the control unit executing processing after receiving the signal generated by the sensor via the external terminal.

### (Supplementary note 36)

The sensor placement method according to supplementary note 35, wherein a variable mechanism that enables at least one of a position and an orientation of the sensor to be changed is attached to the base.

### (Supplementary note 37)

The sensor placement method according to supplementary note 35 or 36, wherein the step (b) comprises partially implanting the sensor within the body at the same time as disposing the base on the skin.

### (Supplementary note 38)

A sensor placement method for placing a sensor that generates a signal according to a state of a substance contained in a body fluid within a body, comprising the steps of
(a) disposing a base on skin, the base being provided with an external terminal that directs the signal generated by the sensor to the outside;
(b) partially implanting the sensor within the body, and causing the sensor to be held by the base; and
(c) attaching a control unit to the base, the control unit executing processing after receiving the signal generated by the sensor via the external terminal.

### (Supplementary note 39)

The sensor placement method according to supplementary note 38, wherein a variable mechanism that enables at least one of a position and an orientation of the sensor to be changed is attached to the base.

### (Supplementary note 40)

The sensor placement method according to supplementary note 38 or 39, wherein the step (b) comprises partially implanting the sensor within the body, and, at the same time, causing the sensor to be held by the base.

Although the invention is described above with reference to embodiments, the invention is not limited to the embodiments. Those skilled in the art will appreciate that various modifications can be made to the configurations and details of the invention without departing from the scope of the invention.

This application is based upon and claims the benefit of priority of prior Japanese Patent Application No. 2009-291706, filed on December 24, 2009, and Japanese Patent Application No. 2010-017723, filed on January 29, 2010, the entire contents of which are incorporated herein by reference.

### Industrial Applicability

As described above, the present invention enables the occurrence of a situation where the function of an embedded sensor is impaired when embedding the sensor under the skin and performing measurement to be suppressed. The present invention is useful in the case of measuring numerical information of a living body continuously such as CGM.

### List of Reference Numerals

- 1: measuring apparatus
- 2: sensor unit (Embodiment 1)
- 3: control unit (Embodiments 1-3)
- 10: base
- 11: variable mechanism (Embodiment 1 : ball joint)
- 11a, 11b: shafts of ball joint
- 12: external terminal
- 13: terminal
- 14: recessed portion
- 15: sensor
- 15a: tip-end portion of sensor
- 16a, 16b: electrodes of sensor
- 17: enzyme reagent layer
- 18: substrate
- 20: sensor unit (Embodiment 2)
- 21: variable mechanism (Embodiment 2)
- 22: rotating member
- 23: holding member
- 23a-23c: portions of holding member
- 24: sensor unit (Embodiment 2)
- 25: variable mechanism (Embodiment 2)
- 26: first holding member
- 26a-26e: portions of first holding member
- 27: protrusions
- 28: second holding member
- 30: sensor unit (Embodiment 3)
- 31: variable mechanism (Embodiment 3)
- 32: rotating member
- 33: holding member
- 33a-33c: portions of holding member
- 34: terminal
- 35: slit
- 36: sensor (Embodiment 3)
- 36a: tip-end portion of sensor
- 36b: base-end portion of sensor
- 37: connection terminal
- 40: skin
- 41: implanting device (Embodiments 1, 2)
- 42: implanting device (Embodiment 3)
- 50: sensor unit (Embodiment 4)
- 51: sensor holding member
- 52: external terminal
- 53: base
- 54: control unit
- 56: recessed portion
- 60: sensor unit (Embodiment 5)
- 61: sensor holding member
- 62: slit
- 63: terminal
- 64: base
- 65: external terminal
- 66: control unit
- 100: measuring apparatus (Embodiment 4)
- 101: measuring apparatus (Embodiment 5)

## Claims

1. A measuring apparatus for measuring numerical information relating to a substance contained in a body fluid within a body, comprising a sensor unit and a control unit,
wherein the sensor unit includes:
a sensor that generates a signal according to a state of the substance;
a base that holds the sensor; and
a variable mechanism that is attached to the base and enables at least one of a position and an orientation of the sensor to be changed, and
the control unit is formed so as to be attachable to the base, and executes processing after receiving the signal generated by the sensor.

2. The measuring apparatus according to claim 1,
wherein the variable mechanism includes a ball joint, and
a shaft at one end of the ball joint is attached to the sensor and a shaft at the other end of the ball joint is attached to the base.

3. The measuring apparatus according to claim 1,
wherein the variable mechanism includes a rotating member that is held in a rotatable state, and
the rotating member is attached to the sensor.

4. A measuring apparatus for measuring numerical information relating to a substance contained in a body fluid within a body, comprising a sensor unit and a control unit,
wherein the sensor unit includes:
a sensor that generates a signal according to a state of the substance;
a base that holds the sensor; and
an external terminal that is provided in the base and directs the signal generated by the sensor to the outside, and
the control unit is formed so as to be attachable to the base, and executes processing after receiving the signal generated by the sensor via the external terminal.

5. The measuring apparatus according to claim 4, wherein the sensor unit further includes a variable mechanism that is attached to the base and enables at least one of a position and an orientation of the sensor to be changed.

6. A sensor placement method for placing a sensor within a body, the sensor generating a signal according to a state of a substance contained in a body fluid within the body, comprising the steps of:
(a) disposing a base on skin, the base being provided with an external terminal that directs the signal generated by the sensor to the outside;
(b) partially implanting the sensor within the body, and causing the sensor to be held by the base; and
(c) attaching a control unit to the base, the control unit executing processing after receiving the signal generated by the sensor via the external terminal.

7. The sensor placement method according to claim 6, wherein a variable mechanism that enables at least one of a position and an orientation of the sensor to be changed is attached to the base.

8. The sensor placement method according to claim 7,
wherein the variable mechanism includes a ball joint, and
a shaft at one end of the ball joint is attached to the sensor and a shaft at the other end of the ball joint is attached to the base.

9. The sensor placement method according to claim 7,
wherein the variable mechanism includes a rotating member that is held in a rotatable state, and
the rotating member is attached to the sensor.

10. The sensor placement method according to any of claims 6 to 9, wherein the step (b) comprises partially implanting the sensor within the body, at the same time as which the base and the sensor become electrically connected.

11. A sensor placement method for placing a sensor within a body, the sensor generating a signal according to a state of a substance contained in a body fluid within the body, comprising the steps of:
(a) disposing a base on skin in a state where the sensor is held by the base, and partially implanting the sensor within the body; and
(b) attaching a control unit to the base, the control unit executing processing after receiving the signal generated by the sensor.

12. The sensor placement method according to claim 11, wherein a variable mechanism that enables at least one of a position and an orientation of the sensor to be changed is attached to the base.

13. The sensor placement method according to claim 12,
wherein the variable mechanism includes a ball joint, and
a shaft at one end of the ball joint is attached to the sensor and a shaft at the other end of the ball joint is attached to the base.

14. The sensor placement method according to claim 12,
wherein the variable mechanism includes a rotating member that is held in a rotatable state, and
the rotating member is attached to the sensor.

15. The sensor placement method according to any of claims 11 to 14,
wherein an external terminal that directs the signal generated by the sensor to the outside is provided in the base,
the control unit includes a terminal that contacts with the external terminal included in the base, and
the step (b) comprises connecting the external terminal provided in the base and the terminal included in the control unit.
